(19) Europäisches Patentamt

European Patent Office

Office européen des brevets

(11) **EP 1 071 022 A2**

## (12) EUROPEAN PATENT APPLICATION

(43) Date of publication:
**24.01.2001 Bulletin 2001/04**

(51) Int. Cl.⁷: **G06F 17/30**

(21) Application number: **00202614.4**

(22) Date of filing: **20.07.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **23.07.1999 US 145407 P**

(72) Inventors:
• **Duckworth, Jianmei F.**
  **King of Prussia, PA 19406 (US)**
• **Stodola, Robert K.**
  **King of Prussia, PA 19406 (US)**

(74) Representative:
**Giddings, Peter John, Dr. et al**
**SmithKline Beecham plc**
**Corporate Intellectual Property,**
**Two New Horizons Court**
**Brentford, Middlesex TW8 9EP (GB)**

(71) Applicant:
**SMITHKLINE BEECHAM CORPORATION**
**Philadelphia Pennsylvania 19103 (US)**

## (54) Systems and methods for generating dataset result matrices

(57) This invention relates to intelligent componentry that can dynamically and incrementally produce result matrices from binary operations such as Blast. By dividing a dataset into same, new, changed and deleted subsets one can determine the minimum operations that need to be done in order to keep result matrices up-to-date with the changes in given datasets.

EP 1 071 022 A2

**Description**

Area of the Invention

Herein is disclosed intelligent componentry that can dynamically and incrementally produce result matrices from binary operations such as Blast. By dividing a dataset into same, new, changed and deleted subsets, one can determine the minimum operations that need to be done in order to keep result matrices up-to-date with the changes in given datasets.

Background of the Invention

The advent of high-throughput sequencing technology and influx of expressed sequence tag/genomic sequences from all over the world pose a great challenge for computational biologists to create and maintain an up to date result matrix for sequence comparison in a timely fashion. New members can be added continuously into two datasets that are involved in the comparison. By the same token, old members can be changed or deleted frequently. Sequence comparisons are, in general, expensive. Limited computer resources often cannot keep up with the demand to refresh result matrices periodically, if sequences have to be processed all over again.

Periodic comparison study which involves vary large datasets can be a daunting task, especially when elements of the datasets are changing, new members are being added and old items are deleted with time. Often, one can not afford to process the datasets from scratch, due to the constraints of time and resources. Thus, an incremental way of data handling is more desirable and cost effective.

Besides, the knowledge of operational symmetry for a given comparison may be needed in order to take advantage of "reverse comparison"—i.e., doing B compare A for A compare B when the former execution is faster than the latter and either way produces the same results that one expected from dataset A and B.

However, one must decide how much update or incremental processing, based on the existing results, is sufficient and yet not redundant, in order to achieve data accuracy and consistency as well as process efficiency.

In addition, one must fully understand the local as well as global variables that determine the result of comparison before an incremental data processing method is deployed. For instance, in Blast search (Altschul & Gish), the measurement of homology/similarity significance, p-value, is not only affected by the pair wise sequence alignment scores and query sequence composition as well as query length (local determinants), but also the scoring matrix and the size of target database (global factors).

Having failed to find a satisfactory method for doing minimum but sufficient incremental comparisons for two changing datasets from literatures, we used biological sequence comparisons as the starting point and developed a way to tell the changes within a dataset from two different aggregation points. We rely on a result matrix system for data storage and as the foundation of minimum incremental comparison that can be apply to any binary operation involving two large datasets, as we present in this paper.

The system disclosed herein uses object oriented design. It also provides smart data/process tracking and a speedy recovery mechanism whenever unforeseen errors in hardware, software, or data occur. It can be used with large datasets and in many applications such as sequence annotation and comparative genomics.

Herein is also described an algorithm for comparisons between sequences within a dataset which occurs often in self-clustering. For system maintainability and extendibility such as to include any binary operations in the future, an object oriented and fault tolerant design that can also utilize maximum parallelism such as multiple processors or LSF (Load Sharing Facility) is implemented and described here.

Summary of the Invention

In one aspect this invention relates to a computer-based method for generating a comparison result matrix from datasets in a database comprising:

a. comparing elements in a first dataset $\underline{A}$ and a second dataset B having the formula $\underline{A}$ op B;

b. updating the first dataset $\underline{A}$ to generate an updated first dataset A;

c. detecting differences between the first dataset $\underline{A}$ and the updated first datasets A wherein $A = \Delta A + \underline{A}$;

d. comparing A with B using the following formula:

$$A \text{ op } B = \underline{A} \text{ op } B + \Delta A \text{ op } B$$

e. determining whether calculation of $\Delta A$ op B is faster than A op B; and

f. using $\Delta A$ op B and the result of step a.) to generate the comparison result matrix of A op B where $\Delta A$ op B is faster than A op B.

Variatoins of this this method are also included within the scope of this invention.

Description of the Figures

Figures 1(a) and 1(b) show result matrices.
Figure 2 is a system flow chart of the process.
Figures 3(a) and 3(b) are matrices for intra-dataset comparisons.
Figure 4 is a diagram of the application of this invention to large datasets.

Methods and Examples

General Description

The operations described herein can be carried out on currently available hardware such as a Compaq or IBM server running Windows NT, a UNIX workstation, or a VAX machine running DEC Alpha operating, to name three current non-limiting examples. A distributed computing environment is perferred as represented by a client/server configuration. The operations can be programmed using pretty much any robust language although C++ was used to run the operations as described herein. Sybase or Oracle data bases are examples of off-the-shelf data bases which have the capacity to handle most data sets for running most operations. Operationally, it is preferred to use local disk space to store intermediate results to avoid expensive network traffic when running under a distributed environment.

The first step of the incremental comparison disclosed herein is to detect what are characterized as data difference of a given dataset at the beginning and the end of a time interval. Typically the interval is one day, one week or a month wherein the beginning of the interval will be yesterday, last week or last month.

The second step is to distinguish operational difference for all comparisons processed by the system. This is necessary if different results are produced with the same comparison operator but different parameters. Blast can be used an exemplary system. For two of the same blastp, if the scoring matrices are different between one blastp run and the other, even if they use the same query set and database, they should be treated as two separate operations in this system.

Once the differences are known one puts the pair of datasets included in the comparison into a matrix-like frame. Then one determines all the possible sub-comparisons that may be required to achieve the same results as if they were done the first time at which these two datasets were compared.

Looking into all the sub-comparisons one selects a subset for the minimal incremental operation from the following list:

1. The composition (or group member) of this subset depends on input data and the properties of comparison like operator symmetry.
2. Partition each sub-comparison selected when feasible.
3. Process each partition in serial or in parallel whenever possible so that only the partition in progress needs to be re-run if there is an unexpected process interruption.
4. Log unsuccessful executions for elements of each partition.
5. Feed back failed entries for re-run once the cause of premature executions is determined.
6. Clean up old results corresponding to changed or deleted elements before merging new results into result repository such as database table.
7. Time stamp both new data and results to alert top layer applications for downstream data analysis.

Detection of data difference

Elements of a sequence dataset stored in a fasta file are divided into changed, new, deleted and same subsets, as compared to the same dataset at the previous aggregation point, say last week. At present classification is based on the differences in sequence length and CRC/checksum of a given sequence in the dataset. Meanwhile offset of each sequence to the beginning of the fasta file is also recorded in order to locate the sequence with little time when needed later on.

For example, if identifications (IDs) in the current dataset do not appear in the old one they are gathered into a new subset. If IDs do occur in the previous set but their corresponding lengths or CRCs or both are different from those in the previous one, they are put into a subset nominated *changed*. If IDs in the previous set no longer exist in the current data, they are saved in a subset marked *deleted*. The rest whose IDs and related lengths as well as CRC are the same as those in the previous set are grouped into same subset.

The system allows users to implement classification method other than sequence length and CRC so long as each element has a unique ID within its dataset.

Distinguishing operational difference

As stated above, even if given datasets are equal, differences in results may not just rely on the specific operator used for the comparison but also depend on the input parameters. Thus in this system one registers both operator and key parameters for a given pair of data comparison. Incremental comparison is carried out only if the datasets, the operator and corresponding parameters are compatible.

For Blast, the registration system will determine if it is the same operation (op) as it was done to the same pair of datasets before, based on if it is blastn, blastp, blastx, tblastn or tblastx and what scoring matrix, high scoring pair (HSP) cutoff score, Z (virtual database length), filter function, gap decay rate, overlap fraction, etc.

Determining sub-comparisons

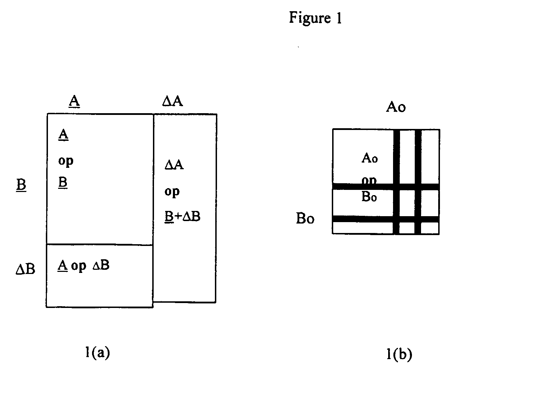

Assume that in the previous run one did a comparison of dataset A against B or AopB for short. But in the current datasets of A and B, one has *new*, *changed* and *deleted* elements as referred to in the previous A and B, i.e., Ao and Bo in Figure 1(b). If one treats changed elements as *deleted* + *new* elements, one then eliminates one group. Now one has *new*, *deleted* and *same* elements

For the *deleted* groups of A and B the system takes out any old results that contain IDs in the *deleted* groups from Ao op Bo as it is shown by the bands in Figure 1(b). Deletions should be done before the new results are loaded into the matrix.

To further illustrate, suppose that the current datasets A and B have elements that are the same as those in the previous set, say $\underline{A}$ and $\underline{B}$, along with new elements which did not exist before, i.e., $\Delta A$ and $\Delta B$. Accordingly $A = \underline{A} + \Delta A$ ; $B = \underline{B} + \Delta B$ . The results of A operation B can sometimes be viewed as the sum of $\underline{A}$ operation $\underline{B}$, $\underline{A}$, operation $\Delta B$, and $\Delta A$ operation $\underline{B} + \Delta B$, as illustrated in Figure 1(a). This may not always be true. It is not the case when elements of dataset comparisons are also affected by some global variables that are different from one dataset collection to another. For instance a Blast p-value is not only determined by its pairwise alignment score but also by the database size which is the size of B. One can set the database size to a fixed value with Z option to get around the problem.

Selecting minimum sub-comparisons

This step is used to determine the minimum calculation needed for A operation B, by examining each sub-comparison group—i.e., $\underline{A}$ operation $\underline{B}$, $\underline{A}$ operation $\Delta B$, and $\Delta A$ operation $\underline{B} + \Delta B$--carefully in conjunction with the properties of the specific comparison operator.

(1) $\underline{A}$ operation $\underline{B}$

This part of re-calculations can be eliminated by using Ao operation Bo if this operator does not depend on any global variables that are different from one collection of A and/or B to the other, such as the size of A or B.

If a complete elimination of $\underline{A}$ operation $\underline{B}$ calculation is impossible, but a brief transformation (operation') can be applied to the prior results ($A_o$ operation $B_o$) to reflect changing variables, then savings from this should also be considered for incremental analysis.

(2) $\underline{A}$ operation $\Delta B$

$\underline{A}$ operation $\Delta B$ should be done with the assumption that operation does not rely on global variables that are affected by the congregation of A and/or B like the size of A or B, or these global variables can be set to be constants between runs.

When the operation is symmetric, i.e. A operation B is the same as B operation A, one should provide an option for allowing the execution of B operation A for A operation B. This can be advantageous time-wise when $\Delta B$ is much smaller than A and, consequently, $\Delta B$ operation A uses much less computer processor time. (3) $\Delta A$ operation $\underline{B}$ + $\Delta B$

Like (1) and (2), this sub-comparison is based on the assumption that the particular operation does not depend on some global variables that are derived from dataset A and differ one dataset collection from another. Or one can bypass it by fixing their values.

Partitioning selected sub-comparison

If a selected sub-comparison contains a long list of elements to be processed, especially if it takes more than a day to run them, it may be better to partition the list into several sublists so that only the sublists in the comparison needs to be re-run in case an unexpected computer software or hardware interruption happens. That is to say, sublists that have already finished the comparison or those waiting to be compared are not affected.

Error log, recovery and time stamp

Unpredicted execution errors in any of the selected sub-comparisons are logged. Dataset elements involved in the failure are saved and resubmitted for next run once the causes are known. Since this process uses the same format for initial data submission and failed entry log, a re-run becomes a re-submission. This circle may go on for a few iterations until all elements have a normal completion.
Speedy recoveries are achieved by partitioning long a to-do list and re-processing only the failed sublists (if any), as mentioned above. Completion time of each comparison is stored along with the results. This way, top layer applications have the choice to retrieve only the most recent results or the combination of both old and new results.

Object Oriented Design and parallelism

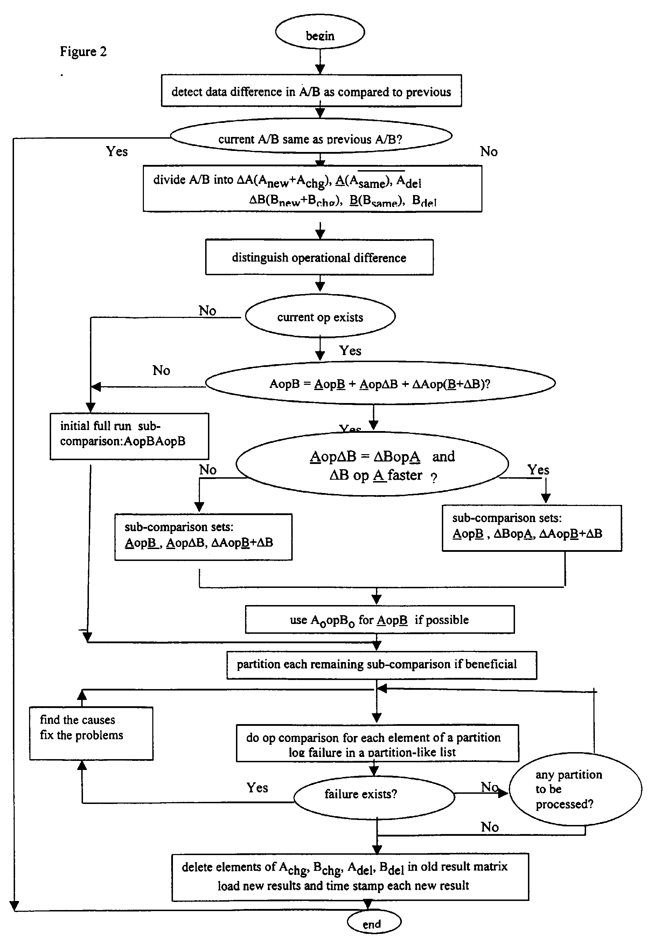

Since the system is meant for increasing the productivity of any binary operation on large datasets, Object Oriented Design is implemented with many C++ features such as virtual, pure virtual and template classes. Default dummy comparison operator can be overridden by operations like blastn, blastp, blastx, Smith Waterman pairwise comparison, etc. The addition of new comparison is almost effortless once the pertinent parser for the comparison is available.
Parallelism can be fully explored after partition, especially if the system is based on a state-machine architecture (Morgart). Different computer processors can run several to-do lists at the same time. Elements in the same to-do list can also be processed in parallel by sending each one to a computer queue managed by a load sharing facility such as LSF (platform computing). This certainly does not rule out the possibility of utilizing distributed system like Common Object Request Broker Architecture (CORBA). System flow chart
All major steps discussed are put together in the flow chart depicted in Figure 2.

Intra-dataset comparisons

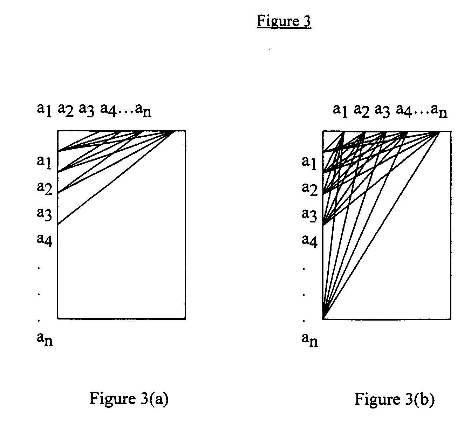

Herein comparisons among elements within a dataset intra-dataset are named *comparisons* or AopA. Take for example dataset A consisting of ($a_1$, $a_2$, $a_3$, $a_4$...$a_n$). In this $a_1$ is often expected to compare with $a_2$, $a_3$, $a_4$... $a_n$. Likewise, $a_2$ compares with $a_3$, $a_4$,...$a_n$ and $a_3$ with $a_4$,...$a_n$.
This AopA differs slightly from AopB, in which B = A. When the same element comparison is not needed, no $a_n$ compares to $a_n$. This AopA can be illustrated with a matrix as in Figure 3(a). One can treat AopA exactly as AopB, where B = A. But this way many comparisons can be redundant as those links shown in Figure 3(b) that are not seen in Figure 3(a).

One constant/stable dataset

When one of the two datasets in a pair-wise comparison has no changes or only alters once a long period of time. One treats the dataset constant until the update occurs at which time one makes the necessary adjustment. If there is more than one dataset constant one may even group them together to do a series of comparison. That is, do comparison cascade ($op_1$, $op_2$ $op_3$,...$op_n$) for one element before processing the other in order to achieve efficiency. To illustrate further, dataset B1, B2,...Bn changes only once a year while dataset A alters every day. Rather than performing A op B1, A op B2, ...and A op Bn it is more advantageous to run A op only, if one can group B1, B2...Bn together and integrate the set into 'op'. When the update of B1,B2,...Bn arrives, figure out the *new*, *changed* and *deleted* of B1, B2, ...Bn. Then, incorporate the new & changed part (ΔB) into 'op' and run A op again to make the data synchronized once more. Deletions for elements in the *deleted* set will be done as shown in Figure 1(b).

Specific Example

Example 1

op = member of blast family

When using blastn, blastp, blastx, tblastn or tblastx to do homology search of sequences in dataset A (query set) against those in dataset B (target database), at minimum, the database size should be set to be the expected length when B is complete with Z option, in order to produce correct results from A, op ΔB and ΔA op B + ΔB for A op B. This is because blast HSP (High-scoring Segment Pair) cutoff score and p-value depend on the total length of dataset B (or B + ΔB) in base pair, according to a study on National Center for Biotechnology Information blast statistics implemented in its version 1.4.8 source code.

Option Y to set query sequence length constant, if one prefers to run ΔB op' A (reverse comparison) and switch the corresponding values of query and target sequences in high scoring segment pair to substitute A op ΔB when ΔB is much smaller than A. For blast is an asymmetric operation. Blast HSP cutoff score is also affected by query length besides database size. Notice that op' is used for ΔB op' A, which means that op and op' may not be the same. In fact, for blastn op' = op; while, on the other hand, op = blastx and op' = tblastn. But setting Z and Y may not be sufficient for reverse comparison since statistic parameter λ may depend on query sequence composition, at least that is the case in Blast1.4.8. Not only that, it has been found that p-value also depends on its pertinent target sequence length. If one changes the op direction from A op B to B op A, that means one switches the query to target and target to query. Thus it is likely to use different length in p-value calculation of B op A than A op B. In other words, one may not get the same p-value as expected from A op B though they may not be significantly different. Of course if one does not care for p-value but only pair-wise alignment score, setting Z and Y and/or other parameters like E, S, T, etc. may do the trick.

It can be useful to calculate p-value of A op B from the results of B op A. This is summarized as follows:

$$sumP = 1 - e^{(-E)}$$

E = f1(Sums, Gdr, dblen, n, Ctx)
Sums = f2(S, r, λ, K, H, m, n, Ovlp)
λ = f3(Qrc, Mx)
H = f4(λ)
K = f5(λ, H)

where

λ is Karlin-Altschul parameter.
K is Karlin-Altschul parameter.
H is Karlin-Altschul parameter for the relative entropy of the target and background residue frequencies.
Qrc is Query sequence residue Composition.
Mx is scoring Matrix like Blosum62, PAM 250...
S stands for a set of pairwise alignment scores, $\{s_1, s_2, ...s_r\}$, corresponding to HSPs that passed overlapping consistency test for sumP calculation.
Grd is Gap decay rate, default 0.5.
Ovlp is Overlap fractions allowed between adjacent HSPs in S, default 1.25.
dblen is number of residues in the target database.
r is number of HSPs in S.
m equals number of residues in Query sequence, e.g. query sequence length for blastn, or query length / 3 for blastx.
n is equal to number of residues in a Target sequence who has matching fragments to Query sequence shown as HSPs.
Ctx refers to Context factor, e.g., 2 for blastn (+,- strand), 6 for blastx (6 frame query sequence translation).
E stands for Expectation.
sumP is Sum probability or p-value.
Sums equals to an aggregated score or sumscore derived from S by function f2.
f1..f5 stand for functions whose value depends on parameters listed within a pair of parenthesis.

From the dependency one can derive p-value (sumP) from pair-wise alignment scores S if one can fix the HSP cut-off score so that S and r are the same no matter whether it is done by A op B or B op' A. Nevertheless the saving from A op ΔB alone can be substantial, especially when ΔB is much smaller than B. This is because, at present, blast program spends a large amount of time on searching homologous sequences that have matching segments to each query sequence in a database. In general the bigger the database the longer the search, and vice versa.

Example 2

op = RepeatMasker

Since RepeatMasker (a sequence masking program invented by Mr Arian Smit and Mr Philllip Green, University of Washington, Fluke Hall on Mason Road, Box 352145, Seattle, WA, 98195, USA.; exemplary URLs -- http://repeatmasker.genome.washington.edu/; http://www.genome.washington.edu/UWGC/analysistools/repeatmask.htm) uses a list of target datasets to process each query sequence and, moreover, these target datasets may only change during a major software update, it seems to be more efficient to treat the target datasets as a constant B as described in A op case. A test was run as follows:

This system was tested by periodically running RepeatMasker on human genomic sequences using A op as well as blastx of these sequences against non-redundant peptides by A op B. Since both human genomic sequence dataset and NR are large and dynamically changing; either RepeatMasker or blastx is computer intensive and the saving of using the described incremental system could be substantial. After the initial run the weekly update on average took only 1/3 of the computational hours used for the first run. Of course that depended on how many *new* or *changed* sequences occurred in the updated dataset A and B as compared to the previous run.

There is some overhead when using this method. The majority of it lies in the data difference detection to calculate sequence CRC, length and offset of each ID to the beginning of the file. Therefore the preferred approach is to use this method for blind full-set recalculation when A and B are large and at least one of them changes frequently, or when each comparison (op) requires more computational power than getting CRC, length and offset, or when each comparison is affected heavily by the size of whole dataset.

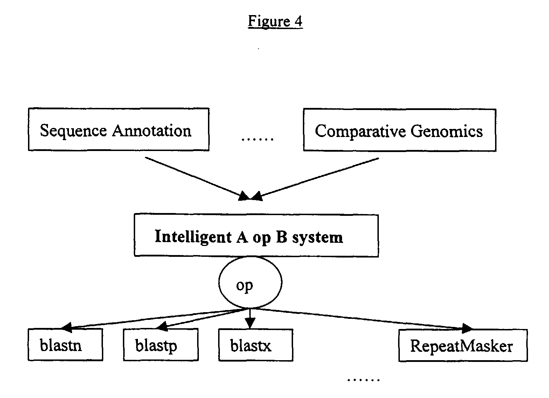

Many applications that deal with large datasets can be built on top of this system. An example is given in Figure 4(e).

**References**

Altschul, S. (1997) Evaluating the Statistical Significance of Multiple Distinct Local Alignments, *Theoretical and Computational Methods in Genome Research*, Plenum Press, New York

Altschul, S. and Madden, T et al. (1997) Gapped BLAST and PSI-BLAST: a new generation of protein database search programs, *Nucleic Acids Research*, vol. 25, 17: 3389-3402

Altschul, S. and Gish, W. (1996) Local Alignment Statistics. *Methods Enzymol*., 266: 460-480.

Altschul, S. et al. (1994) Blast 1.4.8 Source Code, *NCBI*

Altschul, S. Gish, W. Miller, W. Myers, E. and Lipman, D (1990) Basic Local Alignment Search Tool. *J. Mol. Biol.* 215: 403-410

LSF Programmer's Guide, *Platform Computing Corporation* Morgart, W. et al. (1998) State Machine API , *SmithKline Beecham Bioinformatics* Smit, Arian & Green, P.(1999) RepeatMasker Source Code, *Univ. of Washington*

**Claims**

**1.**   A method for generating a comparison result matrix from datasets comprising:

a. comparing elements in a first dataset A and a second dataset B as represented by the formula A, op B;
b. updating the first dataset A to generate an updated first dataset A;
c. detecting differences between the first dataset A and the updated first datasets A wherein A = ΔA + A ;
d. comparing A with B using the following formula:

$$A \text{ op } B = \underline{A} \text{ op } B + \Delta A \text{ op } B$$

e. determining whether calculation of ΔA op B is faster than A op B; and
f. using ΔA op B and the result of step a.) to generate the comparison result matrix of A op B where ΔA op B is faster than A op B.

**2.** A method for generating a comparison result matrix from datasets comprising:

a. comparing elements in a first dataset $\underline{A}$ and a second dataset $\underline{B}$ as represented by the formula $\underline{A}$ op $\underline{B}$;

b. updating the first dataset $\underline{A}$ to generate an updated first dataset A and updating the second dataset $\underline{B}$ to generate an updated second dataset B;

c. detecting differences between the first dataset $\underline{A}$ and the updated first datasets A wherein A = $\Delta$A + $\underline{A}$  and the second dataset $\underline{B}$ and updated second dataset B wherein B = $\Delta$B + $\underline{B}$ ;

d. comparing A with B using the following formula:

$$A \text{ op } B = \underline{A} \text{ op } \underline{B} + (\Delta A \text{ op } (\underline{B} + \Delta B)) + \underline{A} \text{ op } \Delta B;$$

e. determining whether calculation of ($\Delta$A op ($\underline{B}$ + $\Delta$B)) + $\underline{A}$ op $\Delta$B is faster than A op B; and

f. using ($\Delta$A op ($\underline{B}$ + $\Delta$B)) + $\underline{A}$ op $\Delta$B and the result of step a.) to generate the comparison result matrix of A op B where ($\Delta$A op ($\underline{B}$ + $\Delta$B)) + $\underline{A}$ op $\Delta$B is faster than A op B.

**3.** A method for generating a comparison result matrix from datasets comprising:

a. comparing elements in a first dataset $\underline{A}$ and a second dataset $\underline{B}$ as represented by the formula $\underline{A}$ op $\underline{B}$;

b. updating the first dataset $\underline{A}$ to generate an updated first dataset A and updating the second dataset $\underline{B}$ to generate an updated second dataset B;

c. detecting differences between the first dataset $\underline{A}$ and the updated first datasets A wherein A = $\Delta$A + $\underline{A}$  and the second dataset $\underline{B}$ and updated second dataset B wherein B = $\Delta$B + $\underline{B}$ ;

d. comparing A with B using the following formula:

$$A \text{ op } B = \underline{A} \text{ op } \underline{B} + (\Delta A \text{ op } (\underline{B} + \Delta B)) + \Delta B \text{ op } \underline{A} \text{ wherein}$$

$$\Delta B \text{ op } \underline{A} = \underline{A} \text{ op } \Delta B \text{ and } \Delta B \text{ op } \underline{A} \text{ is faster than } \underline{A} \text{ op } \Delta B;$$

e. determining whether calculation of ($\Delta$A op ($\underline{B}$ + $\Delta$B)) + $\Delta$B op $\underline{A}$ is faster than A op B; and

f. using ($\Delta$A op ($\underline{B}$ + $\Delta$B)) + $\Delta$B op $\underline{A}$ and the result of step a.) to generate the comparison result matrix of A op B where ($\Delta$A op ($\underline{B}$ + $\Delta$B)) + $\Delta$B op $\underline{A}$ is faster than A op B.

**4.** The method of claim 1, 2 or 3 wherein the dataset contains biological sequence data.

**5.** A comparison result matrix which is a product of the process of computationally querying datasets wherein the process comprises any one of the processes in any one of claims 1-4.

Figure 1

1(a)

1(b)

Figure 2

```
                          ( begin )
                             │
                             ▼
        ┌────────────────────────────────────────────┐
        │ detect data difference in A/B as compared to previous │
        └────────────────────────────────────────────┘
                             │
                             ▼
Yes   ◄────────  ( current A/B same as previous A/B? )  ────────► No
                             │
                             ▼
        ┌────────────────────────────────────────────┐
        │ divide A/B into ΔA(A_new+A_chg), A̲(A̲same), A_del │
        │        ΔB(B_new+B_chg), B̲(B_same), B_del      │
        └────────────────────────────────────────────┘
                             │
                             ▼
            ┌──────────────────────────────────┐
            │   distinguish operational difference │
            └──────────────────────────────────┘
                             │
                             ▼
No  ◄────────────  ( current op exists )
                             │ Yes
                             ▼
No  ◄───────  ( AopB = A̲opB̲ + A̲opΔB + ΔAop(B̲+ΔB)? )
                             │ Yes
                             ▼
                 ( A̲opΔB = ΔBopA̲  and
                   ΔB op A̲ faster ? )  ──────► Yes
```

initial full run sub-comparison:AopBAopB

No ▼ — sub-comparison sets:
A̲opB̲, A̲opΔB, ΔAopB̲+ΔB

Yes ▼ — sub-comparison sets:
A̲opB̲, ΔBopA̲, ΔAopB̲+ΔB

use $A_0opB_0$ for A̲opB̲  if possible

partition each remaining sub-comparison if beneficial

find the causes
fix the problems

do op comparison for each element of a partition
log failure in a partition-like list

Yes ◄──── ( failure exists? ) ────► No

any partition
to be
processed?

No

delete elements of $A_{chg}$, $B_{chg}$, $A_{del}$, $B_{del}$ in old result matrix
load new results and time stamp each new result

( end )

Figure 3

$a_1$ $a_2$ $a_3$ $a_4$...$a_n$

$a_1$

$a_2$

$a_3$

$a_4$

.

.

.

$a_n$

$a_1$ $a_2$ $a_3$ $a_4$...$a_n$

$a_1$

$a_2$

$a_3$

$a_4$

.

.

.

$a_n$

Figure 3(a)

Figure 3(b)

Figure 4

```
┌─────────────────────────┐                    ┌─────────────────────────┐
│   Sequence Annotation   │    ......          │   Comparative Genomics  │
└─────────────────────────┘                    └─────────────────────────┘
```

Intelligent A op B system

op

blastn    blastp    blastx        RepeatMasker

......